# EUROPEAN PATENT APPLICATION

(11) **EP 1 248 111 A2**
(43) Date of publication of application: **09.10.2002**
(21) Application number: 02290721.6
(22) Date of filing: 21.03.2002
(51) Int. Cl.: G01N 33/543, G01N 33/74

(54) **Kit and method for diagnosing non-pregnancy**

(30) Priority: 04.04.2001 KR 2001017863
(71) Applicant: KOREA GREEN CROSS CORPORATION, Yongin, Kyounggi-do 449-900 (KR); Republic of Korea, Suwon-shi, Gyunggi-do (KR)
(72) Inventor: Seong, Hwan-hoo, Seoul (KR); Cho, Min, Anyang-shi, Gyunggi-do (KR); Won, Yoo-deok, Yongin-shi, Gyunggi-do (KR); Kim, In-wook, Sungnam-shi, Gyunggi-do (KR)
(74) Representative: Ulmann, Catherine Claire

(57) **Abstract**

The present invention relates to a kit for diagnosing non-pregnancy and a method for diagnosing non-pregnancy of an animal using the same, and more particularly, to a kit for diagnosing non-pregnancy of animal comprising: an assay strip, in the form of porous membrane paper comprising a plurality of holes for moving a test sample, wherein progesterone-BSA conjugate is immobilized on one end portion as a test line and anti-IgG is immobilized on the other end portion as a control line; and anti-progesterone IgG-gold conjugate for reacting with the test sample such as blood or milk. The inventive kit for diagnosing non-pregnancy can test the non-pregnancy of milch cow without any special device about 20 days after the artificial insemination. Furthermore, more accurate result can be obtained by using the kit since the non-pregnancy diagnosis is performed by comparing color of the test line with that of the control line.

## Description

### BACKGROUND OF THE INVENTION

### 1. Field of the Invention

The present invention relates to a kit for diagnosing non-pregnancy and a method for diagnosing non-pregnancy of animal using the same, and more particularly, to a kit for diagnosing non-pregnancy and a method for early and easy diagnosis of non-pregnancy by detecting concentration of progesterone in blood, milk etc. of the animal.

### 2. Description of the Related Art

The estrus cycle of a normal milch cow is 19-22 days, and the progesterone concentration in blood or milk of the cow is almost 0 on estrus day. The progesterone concentration increases day by day from the estrus day, and generally decreases to below 1ng/ml on 19th day after the estrus day if the cow is non-pregnant. On the other hand, if the milch cow is pregnant, the progesterone concentration does not decrease. Accordingly, non-pregnancy of milch cow can be examined by measuring progesterone concentration of crude milk or blood between 19th day and 22nd day after the estrus day.

Before 1990s, pregnancy of milch cow was examined through a rectal examination by an expert when 50 or 60 days have passed after artificial insemination or mating. However, such a method generally requires a skilled veterinarian and causes quite a big economical loss because a long time is required to confirm the non-pregnancy of milch cow. In addition, if an ovary or an embryo is spurred through the rectal examination for the pregnancy diagnosis, there is a possibility of an ovarian disease or abortion, and the milch cow can get stressed out. Further, according to the method mentioned above, the non-pregnancy can be doubtlessly diagnosed 50-60 days after the artificial insemination. If the milch cow is examined as not to be pregnant at this moment, the process to catch the estrus sign of the non-pregnant animal and the artificial insemination should be repeated, which results to great economical loss.

Accordingly, since the 1990s, methods to diagnose in a simple and easy manner on the pregnancy, or the breeding trouble of the milch cow are successively being developed, for instance an example would be the method for diagnosing pregnancy/non-pregnancy with a supersonic diagnosing device. However, since there are many cases where the non-pregnancy diagnosis are taken place in a rough field by an amateur, the supersonic diagnosing device needs to be made extremely firm and secure. Thus there is a disadvantage that the device for this method is very large and heavy to be used in the field. Afterwards, an ELISA kit for diagnosing pregnancy of a cow by detecting the concentration of progesterone in blood was developed, but it is also difficult to use the kit in the field since an expensive and delicate analysis device is required and the diagnosing process is complicated.

To overcome such disadvantages and to diagnose non-pregnancy in a simple manner, a method using an assay strip was developed by Laitinen et al.(Acta Chemica Scandinavia, 1996: 50: 141-145). The assay strip is designed so that monoclonal antibody to progesterone is fixed on the nitrocellulose membrane, and the test sample that is obtained from cow(such as milk and blood) moves through the membrane by a capillary attraction. And then progesterone-ovalbumin conjugate in which colloidal gold particles are combined(hereinafter, 'progesterone gold conjugate') moves through the membrane.
At this point, if the concentration level of the progesterone in the test sample is high, the progesterone first combines with the monoclonal antibody to progesterone and the progesterone gold conjugate can not combine with the monoclonal antibody to progesterone not to cause color change of assay strip. On the contrary, when the concentration level of the progesterone in the test sample is low, only a few monoclonal antibody to progesterone combines with the progesterone in the test sample and the progesterone gold conjugate moving later combine with the monoclonal antibody to progesterone to cause color change of assay strip. Accordingly, the pregnancy of cow can be examined by observing the color change of the assay strip or using a chromatography scanner.

However, the disadvantages of the aforementioned method is that since the test sample and progesterone gold conjugate each have to be separately moved through the assay strip for the analysis of the pregnancy/non-pregnancy, the operation procedure can become complicated. Also, when the cow is pregnant, i.e. when the concentration level of progesterone in the test sample is high, even after the completion of the diagnosis, there is no change on the color of the assay strip, and the error due to the defect of the assay strip itself cannot be prevented. In addition, observing from the naked eyes, since there is no objective criterion to judge the pregnancy/non-pregnancy from the change of the color, the problem is that each observant needs to rely on his own judgment.

### SUMMARY OF THE INVENTION

In order to overcome such disadvantages, an object of the present invention is to provide an improved kit for diagnosing non-pregnancy and a simple and easy method for diagnosing non-pregnancy of a cow, such as a milch cow about 20 days after the insemination by examining the progesterone concentration in blood or milk.
Another object of the invention is to provide a kit for diagnosing non-pregnancy and a method for diagnosing non-pregnancy using the same preventing error in diagnosis due to the defect of the assay strip.
Another object of the invention is to provide a kit for diagnosing non-pregnancy and a method for diagnosing non-pregnancy using the same having a high reliability in diagnosis of non-pregnancy.

To achieve the objects as mentioned above, the present invention provides a kit for diagnosing non-pregnancy of animal comprising: an assay strip, in the form of a of porous membrane paper comprising a plurality of holes for moving a test sample, wherein progesterone-BSA conjugate is immobilized on one end portion as a test line and, preferably, anti-IgG is immobilized on the other end portion as a control line; and anti-progesterone IgG-gold conjugate for reacting with the test sample such as blood or milk.
The present invention also provides a method for diagnosing non-pregnancy of animal comprising steps of: mixing anti-progesterone IgG-gold conjugate with a test sample; dipping the end portion, where a test line is formed, of the porous membrane paper of which a progesterone-BSA conjugate is immobilized on one end portion as the test line and anti-IgG is immobilized on the other end portion as a control line, in the mixed solution; and diagnosing non-pregnancy by comparing the color of the test line and the control line.

### BRIEF DESCRIPTION OF THE DRAWINGS

A more complete appreciation of the invention, and many of the attendant advantages thereof, will be readily apparent as the same becomes better understood by reference to the following detailed description when considered in conjunction with the accompanying drawings, wherein:
Figure 1 is a plan view of an assay strip forming a kit for diagnosing non-pregnancy according to an embodiment of the present invention; and
Figure 2 is a drawing for explaining the principle of color generation of the kit for diagnosing non-pregnancy according to an embodiment of the present invention.

### DETAILED DESCRIPTION OF THE INVENTION

For a better understanding of the present invention, reference will now be made in detail to the following disclosures, drawings and appended claims.
The inventive kit for diagnosing non-pregnancy is devised from the fact that, about 20 days after the artificial insemination of the cow on estrus day, progesterone concentration in serum, blood plasma, or milk does not decrease when the cow is pregnant and, on the contrary, the progesterone concentration considerably decreases.

Accordingly, the inventive kit for diagnosing non-pregnancy comprises anti-progesterone IgG-gold conjugate to be mixed with test sample such as serum, blood plasma, or milk of a subject to be examined such as cow or milch cow, and an assay strip to examine the progesterone concentration in the test sample by the naked eye. According to the present invention, non-pregnant period of a subject can be reduced by immediate artificial insemination when the cow is not pregnant.

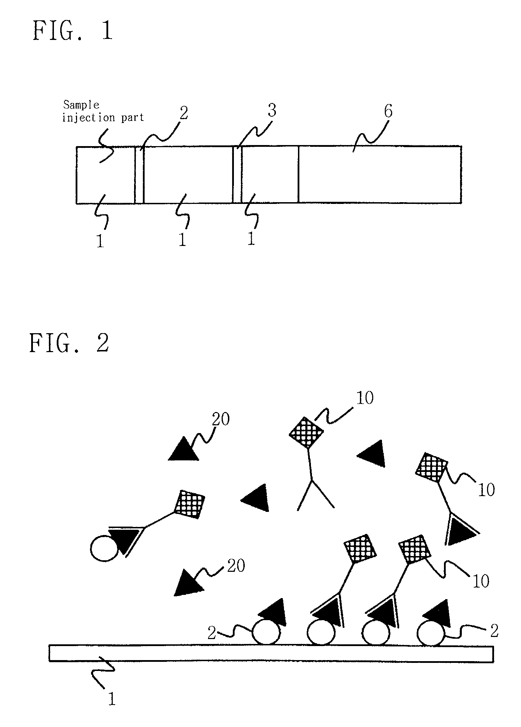

Figure 1 is a plan view of an assay strip forming a kit for diagnosing non-pregnancy according to an embodiment of the present invention. As shown in Figure 1, the assay strip having strip shape comprises a porous membrane paper 1 in which test sample moves from one end to the other end by the capillary attraction and an absorbing paper 6 which is combined with the porous membrane paper 1 and provided for absorbing liquid test sample passing through the porous membrane paper 1.
The size of the assay strip is not limited and can be varied if a predetermined amount of test sample can move through on it, and reaction of the immobilized materials with a target material (analyte) in test sample can occur, and therefore the reaction results can be observed with the naked eyes, but 4mm×50mm is preferable. Further, the porous membrane paper 1 preferably comprises numerous minute holes having preferably 5-12µm of diameter to easily absorb and move the liquid test sample such as serum, blood plasma, or milk with the anti-progesterone IgG-gold conjugate.
In case that the diameter of the minute hole is less than 5µm, the assay strip is not practical since the absorbing and moving speed of the test sample is too slow. On the contrary, in case that the diameter of the minute hole is greater than 12µm, the diagnosis of pregnancy may be inaccurate since the moving speed of the test sample is too fast. Thus, the size of the minute hole can be properly controlled according to the desired moving speed of the test sample and the desired time required for the diagnosis. The material of the porous membrane paper 1 is not particularly restricted. Preferably, the porous membrane paper 1 is formed of nitrocellulose membrane, and the absorbing paper 6 is formed of cellulose paper.
On one portion of the porous membrane paper 1, progesterone-BSA conjugate 2 is immobilized as a test line apart from a distal end of the porous membrane paper 1 by a predetermined distance, preferably 10mm. On the other portion distanced from the test line of progesterone-BSA conjugate area 2 by a predetermined distance, anti-IgG 3 is immobilized as a control line. The anti-IgG immobilized as a control line is preferably anti-IgG obtained from one species of animal selected from the group consisting of mouse, goat, and sheep. And more preferable anti-IgG is produced by immunizing IgG derived from the same species of animal which IgG of the anti-progesterone IgG-gold conjugate is obtained.

The anti-progesterone IgG-gold conjugate consisting the inventive kit for diagnosing pregnancy is selected to react with the anti-IgG of the control line. Preferably, anti-progesterone mouse IgG-gold conjugate is used and generally permissible stabilizer can be used if required. For example, non-restrictively, BSA, sucrose, etc can be used as the stabilizer. The amount of stabilizer can be varied according to the type of stabilizer, but 1-5weight% of total amount of diagnosis reagent is preferable. The anti-progesterone IgG-gold conjugate including the stabilizer can be manufactured by freeze drying. In use, the freeze dried conjugate is dissolved in a buffer solution of phosphoric physiological salt containing non-ionic surfactant such as 1% Tween 20(Hayashi Purechemical Industries Ltd., Osaka), and then mixed with serum, blood plasma, or milk of a subject to be examined such as cow or milch cow, and used as a reagent for diagnosing non-pregnancy.

In operation, one drop(0.04mL) of test sample such as serum, blood plasma, or milk obtained from subject to be examined such as cow or milch cow which was inseminated 19 or 21 days ago is dropped into a drop(about 0.025mL) of anti-progesterone IgG-gold conjugate solution made by dissolving the conjugate with a predetermined amount of buffer solution. The test sample and the conjugate are mixed together; and then the distal end of the assay strip(sample injection part) is dipped into the mixed solution.

When the assay strip is dipped into the mixed solution, solution moves to the upper portion along the assay strip by the capillary attraction, and then passes through the test line of progesterone-BSA conjugate layer 2. At this moment, anti-progesterone IgG-gold conjugate in the mixed solution is combined with the progesterone-BSA conjugate in the test line 2 by the antigen-antibody reaction, which results in color change.

Figure 2 is a drawing for illustrating the principle of color generation of the kit for diagnosing non-pregnancy according to the present invention. As shown in Figure 2, a progesterone-BSA conjugate layer 2 is formed on the upper side of the porous membrane paper 1 of the assay strip. When the reagent for diagnosis containing the anti-progesterone IgG-gold conjugate 10 passes through the conjugate layer 2, the anti-progesterone IgG-gold conjugate 10 is reacted and combined with the layer of the progesterone-BSA conjugate 2.

In case that the cow to be examined is pregnant, the concentration of progesterone 20 increases to more than 5ng/ml, and most of the anti-progesterone IgG-gold conjugate 10 is mixed and combined with the progesterone 20 contained in the test sample, and therefore little anti-progesterone IgG gold conjugate binds with the progesterone-BSA conjugate layer 2 formed on the porous membrane paper 1. Accordingly, the color generation of test line 2 is paler than that of the control line. On the contrary, in case that the cow is not pregnant, the concentration of progesterone 20 decreases to 1ng/ml, and most of anti-progesterone IgG-gold conjugate 10 is combined with the progesterone-BSA conjugate layer 2 formed on the porous membrane paper 1. Accordingly, the color generation of test line 2 is stronger than that of control line. As mentioned above, the comparison of color generations of the test line 2 and the control line 3 allows easier diagnosis of non-pregnancy/pregnancy.

When progesterone 20 in blood is mixed with anti-progesterone IgG-gold conjugate 10 and then reaches to the position 2 where progesterone-BSA is immobilized by capillary attraction, the progesterone 20 in the test sample and the progesterone-BSA immobilized on the thin film 1 will competitively be combined with the anti-progesterone IgG-gold conjugate 10, and therefore, the degree of scarlet of test line 2 is varied in inverse proportion to the progesterone content in the test sample. That is, non-pregnancy of a subject can be diagnosed by verifying the color generation degree of the progesterone-BSA conjugate layer 2 with the naked eye.

The anti-progesterone IgG-gold conjugate passing through the progesterone-BSA conjugate layer 2 is detected on the control line on which sheep anti-mouse IgG 3 is fixed, which results in the color change of the control line 3. The amount of anti-progesterone IgG-gold conjugate combined to the sheep anti-mouse IgG is in direct proportion to the amount of progesterone in the test sample.

Accordingly, the user(owner of cow) can diagnose non-pregnancy of the animal by comparing the colors of the test line of progesterone-BSA conjugate layer 2 and the control line where the anti-mouse antibody 3 is immobilized. When the color of test line 2 is paler than that of control line, the animal is judged as pregnant.

The following table 1 shows the pregnancy diagnosis of Korea-grown cow on the 19th, 20th, and 21st day after the artificial insemination by using the inventive kit for diagnosing non-pregnancy.

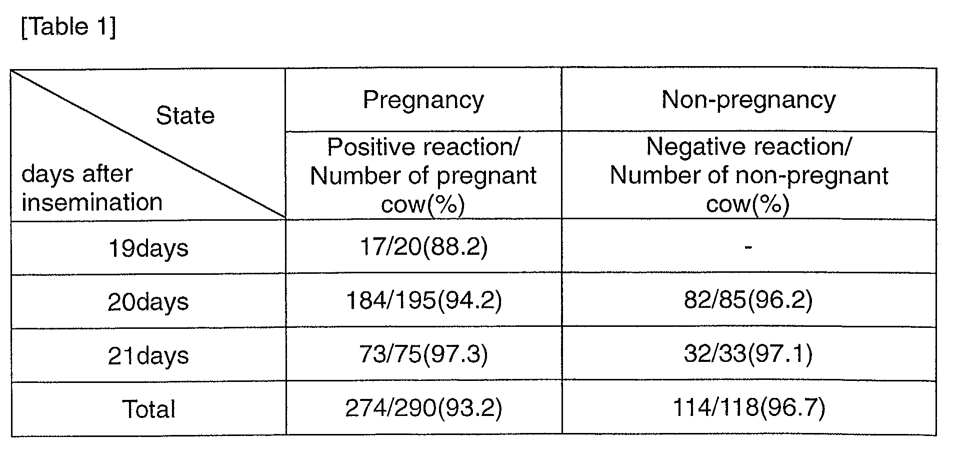

As shown in Table 1, the inventive kit for diagnosing non-pregnancy has 93.2% accuracy in case of pregnancy diagnosing test and 96.7% of accuracy in case of non-pregnancy diagnosing test. Accordingly, the kit for diagnosing non-pregnancy of the present invention allows pregnancy/non-pregnancy diagnosis with high accuracy and especially accurate in non-pregnancy diagnosis. That is, the inventive kit for diagnosing non-pregnancy has superior economical advantages since the user can accurately select the non-pregnant animal after artificial insemination and reduce non-pregnant period.

The kit for diagnosing non-pregnancy according to the present invention can diagnose the pregnancy/non-pregnancy, especially non-pregnancy of the cow such as Korea-grown cow, milch cow, or Japan-grown cow with the concentration of progesterone in blood or milk. Since the inventive kit for diagnosing non-pregnancy has a control line which can be compared with a test line, the defect of assay strip can be verified and the non-pregnancy can be objectively and very accurately diagnosed.

## Claims

1. A kit for diagnosing non-pregnancy of animal comprising:
an assay strip, in the form of of porous membrane paper comprising a plurality of holes for moving a test sample, wherein progesterone-BSA conjugate is immobilized on one end portion as a test line and anti-IgG is immobilized on the other end portion as a control line; and
anti-progesterone IgG-gold conjugate for reacting with the test sample.

2. A kit for diagnosing non-pregnancy according to claim 1, wherein the IgG of the control line is obtained from one species of animal selected from the group consisting of mouse, goat, and sheep.

3. A kit for diagnosing non-pregnancy according to claim 1, wherein the anti-progesterone IgG-gold conjugate is used by being dissolved in a buffer solution of phosphoric physiological salt containing non-ionic surfactant.

4. A kit for diagnosing non-pregnancy according to claim 1, wherein the plurality of holes has a diameter of 5-12µm.

5. A method for diagnosing non-pregnancy of an animal comprising steps of:
mixing anti-progesterone IgG-gold conjugate with a test sample;
dipping the end portion, where a test line is formed, of the porous membrane paper of which a progesterone-BSA conjugate is immobilized on one end portion as the test line and anti-IgG are immobilized on the other end portion as a control line, in the mixed solution; and
diagnosing non-pregnancy by comparing the color of the test line and the control line.

6. A method for diagnosing non-pregnancy of animal according to claim 7, wherein the progesterone in the test sample and the progesterone of the progesterone-BSA conjugate are competitively reacted to the anti-progesterone mouse IgG-gold conjugate on the test line.

7. A kit for diagnosing non-pregnancy of animal comprising:
an assay strip, in the form of porous membrane paper comprising a plurality of holes for moving a test sample, wherein progesterone-BSA conjugate is immobilized on one end portion as a test line; and
anti-progesterone IgG-gold conjugate for reacting with the test sample.
